# EUROPÄISCHE PATENTSCHRIFT

(11) **EP 2 539 314 B1**
(45) Veröffentlichungstag und Bekanntmachung des Hinweises auf die Patenterteilung: **08.04.2015**
(21) Anmeldenummer: 11704624.3
(22) Anmeldetag: 23.02.2011
(51) Int. Cl.: C07C 263/10, C07C 265/14

(54) **VERFAHREN ZUR HERSTELLUNG VON ISOCYANATEN IN DER GASPHASE**
METHOD FOR PRODUCING ISOCYANATES IN THE GAS PHASE
PROCÉDÉ POUR LA PRODUCTION D'ISOCYANATES DANS LA PHASE GAZEUSE

(30) Priorität: 26.02.2010 EP 10154861
(43) Veröffentlichungstag der Anmeldung: 02.01.2013
(73) Patentinhaber: BASF SE, 67056 Ludwigshafen (DE)
(72) Erfinder: MATTKE, Torsten, 67251 Freinsheim (DE); BOEHLING, Ralf, 64653 Lorsch (DE); KNOESCHE, Carsten, 67150 Niederkirchen (DE); LEHR, Vanessa, Simone, 68199 Mannheim (DE)
(86) Internationale Anmeldenummer: PCT/EP2011/052658
(87) Internationale Veröffentlichungsnummer: WO 2011/104264

(56) Entgegenhaltungen:
- EP-A1- 1 555 258
- EP-A2- 1 319 655
- EP-A2- 1 754 698

## Beschreibung

Die Erfindung betrifft ein Verfahren zur Herstellung von Isocyanaten durch Umsetzung der korrespondierenden Amine mit Phosgen in der Gasphase, gegebenenfalls in Gegenwart eines Inertmediums, das folgende Schritte umfasst:
(a) Verdampfen des Amins in einem Verdampfer,
(b) Überhitzen des Amins,
(c) Mischen des gasförmigen Amins mit dem Phosgen und Einleiten in eine Reaktionszone,
(d) Umsetzen von Amin und Phosgen in der Reaktionszone zum Isocyanat, wobei ein Isocyanat und ein Chlorwasserstoff enthaltendes Reaktionsgemisch gebildet wird,
(e) Abkühlen des Isocyanat und Chlorwasserstoff enthaltenden Reaktionsgemischs.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität möglich und ein geringerer Hold-Up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils im gasförmigen Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 beschrieben.

Um Folgereaktionen zu vermeiden, wird das Reaktionsgemisch nach Reaktionsende schnell abgekühlt. Hierzu wird zum Beispiel ein Flüssigkeitsquench eingesetzt.

Um das Amin zu verdampfen und auf Reaktionstemperatur von über 300°C zu überhitzen, werden derzeit im Wesentlichen elektrische Beheizung, Verbrennungsgase oder unter hohem Druck stehender Wasserdampf eingesetzt. Gegebenenfalls wird auch eine Salzschmelze als Wärmeträgermedium verwendet. Während der Verdampfung und Überhitzung des Amins bei hohen Temperaturen kann es zu Zersetzungs- und Oligomerisierungsreaktionen kommen. Dies führt zum einen zu Ausbeuteverlusten des Gesamtprozesses, zum anderen können gebildete höhere Oligomere unverdampft bleiben und zu Ablagerungen im Verdampfer und in dem Verdampfer folgenden Anlageteilen führen. Um Ausbeuteverluste und Verstopfungsgefahr zu minimieren ist es notwendig, schnelle Verdampfungs- und Überhitzungszeiten für das Amin einzuhalten. Diese werden im Wesentlichen durch hohe volumenspezifische Wärmetauscherflächen erreicht. Als volumenspezifische Wärmetauscherfläche wird dabei der Quotient aus wärmeübertragender Oberfläche und dem durch das zu verdampfende Amin durchströmten Volumen definiert. Ein Verdampfer, bei dem das Amin durch Kanäle geführt wird und eine spezifische Wärmetauscherfläche von 1000 m²/m³ bereitgestellt wird, ist aus EP-A 1 754 698 bekannt. Nachteil des beschriebenen Verdampfers ist jedoch, dass eine räumliche Trennung der Strömungspfade in den einzelnen Kanälen vorliegt. Wenn es zu Ablagerungen in einem Kanal kommt, wird dieser zunächst weniger durchströmt, wodurch sich die Verweilzeiten in dem Kanal erhöhen, wodurch sich Zersetzungen des Amins mit weiteren Ablagerungen verstärken. Dies führt zu einer Verstopfung des Kanals. Die Wärmetauscherfläche dieses Kanals steht somit nicht mehr zur Verdampfung zur Verfügung. Bei gleichbleibendem Volumenstrom werden gleichzeitig die anderen, verbleibenden Kanäle stärker durchströmt, woraus erhöhte Druckverluste und kürzere Verweilzeiten resultieren, was gegebenenfalls dazu führt, dass das Amin nicht vollständig verdampft. Der Einsatz paralleler Kanalstrukturen, die von dem Amin durchströmt werden, ist daher sehr sensitiv auf Ablagerungen in einzelnen Kanälen. Ein weiteres Problem, das sich beim Durchströmen des Amins durch einzelne Kanäle ergibt, ist eine gleichmäßige Verteilung des Amins auf alle Kanäle.

Aufgabe der vorliegenden Erfindung ist es daher, ein Verfahren zur Herstellung von Isocyanaten durch Umsetzung der korrespondierenden Amine mit Phosgen in der Gasphase bereitzustellen, bei dem das Amin in einem Verdampfer mit hoher spezifischer Wärmetauscherfläche und damit kurzen Verdampfungs- und Überhitzungszeiten verdampft wird, das die Nachteile der aus dem Stand der Technik bekannten Verdampfer nicht aufweist.

Gelöst wird die Aufgabe durch ein Verfahren zur Herstellung von Isocyanaten durch Umsetzung der korrespondierenden Amine mit Phosgen in der Gasphase, gegebenenfalls in Gegenwart eines Inertmediums, das folgende Schritte umfasst:
(a) Verdampfen des Amins in einem Verdampfer,
(b) Überhitzen des Amins,
(c) Mischen des gasförmigen Amins mit dem Phosgen und Einleiten in eine Reaktionszone,
(d) Umsetzen von Amin und Phosgen in der Reaktionszone zum Isocyanat, wobei ein Isocyanat und ein Chlorwasserstoff enthaltendes Reaktionsgemisch gebildet wird,
(e) Abkühlen des Isocyanat und Chlorwasserstoff enthaltenden Reaktionsgemischs,
wobei der Verdampfer einen Behälter umfasst, in dem Rohre enthalten sind, die von einem Heizmedium durchströmt werden, wobei Anzahl und Durchmesser der Rohre so ausgeführt sind, dass die Rohre eine spezifische Wärmetauscherfläche bezogen auf das vom Amin durchströmte Volumen von mindestens 300 m²/m³ aufweisen.

Als spezifische Wärmetauscherfläche im Rahmen der vorliegenden Erfindung ist die vom Amin umströmte Oberfläche sämtlicher im Verdampfer enthaltener, von Heizmedium durchströmter Rohre bezogen auf das vom Amin durchströmte Volumen zu verstehen.

Vorteil der Durchströmung der Rohre des Verdampfers mit dem Heizmedium und des Umströmens der Rohre mit dem Amin ist, dass die Strömungspfade des Amins nicht in einzelne Kanäle aufgeteilt werden. Zudem wird aufgrund der vergleichsweise großen zusammenhängenden, von dem Amin durchströmten Fläche vermieden, dass durch sich gegebenenfalls bildende Ablagerungen die Zersetzung des Amins und damit die Bildung weiterer Ablagerungen beschleunigt wird. Die Ausbildung von geringen Ablagerungen führt nur zu einer minimalen Erhöhung des Druckverlusts und damit auch zu zunächst unmerklichen Verkürzungen der Verweilzeit. Der Verdampfer ist somit auch bei Ausbildung von Ablagerungen zunächst noch ohne Einschränkungen einsetzbar. Zudem führt die unverändert hohe Strömungsgeschwindigkeit des Amins auch bei Ausbildung geringer Ablagerungen dazu, dass die Ablagerungen zumindest teilweise mit dem Aminstrom mitgerissen werden und somit die Gefahr der Bildung von Ablagerungen weiter reduziert wird.

Um eine schnelle Verdampfung und gegebenenfalls Überhitzung des Amins zu erzielen, wird eine große spezifische Wärmetauscherfläche gewählt. Die Wärmetauscherfläche beträgt erfindungsgemäß mindestens 300 m²/m³. Bevorzugt ist die spezifische Wärmetauscherfläche bezogen auf das vom Amin durchströmte Volumen mindestens 400 m²/m³ und insbesondere mindestens 500 m²/m³.

Um eine schnelle Verdampfung des Amins zu erzielen und weiterhin die Ausbildung von Ablagerungen an den Wärmetauscherflächen zu vermeiden, ist es weiterhin bevorzugt, dass die Rohre parallel zur Strömungsrichtung des Amins im Behälter angeordnet sind. Hierbei können die Rohre entweder im Gleichstrom zum Amin oder im Gegenstrom zum Amin vom Heizmedium durchströmt werden. Bevorzugt ist eine Durchströmung der Rohre mit dem Heizmedium im Gegenstrom.

Um weiterhin die Ausbildung von Ablagerungen an den Oberflächen der Rohre zu reduzieren, ist es in einer Ausführungsform der Erfindung bevorzugt, wenn die Rohre eine glatte Oberfläche aufweisen. Unter glatter Oberfläche wird dabei insbesondere eine Oberfläche ohne Rippen oder sonstige Erhebungen und ohne Einkerbungen verstanden. Weiterhin ist auch eine geringe Rauigkeit der Oberfläche der Rohre vorteilhaft. Je geringer die Rauigkeit der Oberfläche ist, umso weniger Keime befinden sich an der Oberfläche, an denen sich Ablagerungen anlagern können.

Zur Erzielung einer gleichmäßigen Umströmung der Rohre mit dem Amin ist es weiterhin bevorzugt, wenn die Rohre einen kreisförmigen Querschnitt aufweisen. Neben einem kreisförmigen Querschnitt ist jedoch auch jeder beliebige andere Querschnitt möglich. Vorteil eines kreisförmigen Querschnitts ist, dass die Rohre hierbei keine Kanten aufweisen, an denen sich Ablagerungen bilden können. Zudem ermöglichen Rohre mit einem kreisförmigen Querschnitt den Einsatz einer großen Anzahl an Rohren und damit einer großen spezifischen Wärmetauscherfläche.

Um die spezifische Wärmetauscherfläche bezogen auf das vom Amin durchströmte Volumen von mindestens 300 m²/m³ zu erhalten, werden im Verdampfer vorzugsweise Rohre mit einem Außendurchmesser von maximal 10 mm, bevorzugt von maximal 8 mm eingesetzt. Der kleinste Abstand zwischen zwei benachbarten Rohren liegt vorzugsweise bei maximal 3 mm. Der Rohrdurchmesser von maximal 10 mm und der Abstand zwischen zwei Rohren von maximal 3 mm führt zu einer hohen spezifischen Wärmetauscherfläche bezogen auf das vom Amin durchströmte Volumen.

Die für die Verdampfung und gegebenenfalls Überhitzung des Amins notwendige Verweilzeit wird durch die entsprechende Länge des Verdampfers und damit der Rohre sowie den den Verdampfer durchströmenden Volumenstrom eingestellt. Je höher der Volumenstrom bei gleichbleibender vom Amin durchströmter Fläche ist, umso höher ist die Geschwindigkeit und damit umso kürzer die Verweilzeit des Amins im Verdampfer. Um bei gleichbleibender vom Amin durchströmter Querschnittsfläche und gleichbleibendem Volumenstrom eine Verlängerung der Verweilzeit zu erhalten, ist es notwendig, die Rohre und damit den Verdampfer länger zu gestalten. Für einen zur Verdampfung und gegebenenfalls Überhitzung von Amin eingesetzten Verdampfer ist die Länge der Rohre vorzugsweise im Bereich von 0,1 bis 5 m, mehr bevorzugt im Bereich von 0,2 bis 3 m und insbesondere im Bereich von 0,3 bis 2 m.

Neben dem Verdampfen des Amins in dem Verdampfer ist es weiterhin auch möglich, das Amin dem Verdampfer mit einer Temperatur unterhalb der Verdampfungstemperatur zuzuführen und im Verdampfer zunächst auf Verdampfungstemperatur vorzuheizen. Nach dem Erreichen der Verdampfungstemperatur wird das Amin im Verdampfer verdampft. Da die Reaktionstemperatur zur Umsetzung des Amins mit Phosgen zum korrespondierenden Isocyanat oberhalb der Verdampfungstemperatur des Amins liegt, üblicherweise im Bereich von 300 bis 400°C, ist es notwendig, das Amin nach dem Verdampfen zu überhitzen. Das Überhitzen des Amins erfolgt vorzugsweise ebenfalls im Verdampfer.

Der Druck, bei dem das Amin in dem Verdampfer verdampft wird, liegt vorzugsweise im Bereich von 0,05 bis 10 bar abs. Besonders bevorzugt liegt der Druck im Bereich von 0,8 bis 5 bar abs.

Nach dem Überhitzen wird das Amin mit dem Phosgen gemischt. Hierbei liegt die Temperatur des Phosgens vorzugsweise im Bereich von 250 bis 450°C. Die Erwärmung des Phosgens kann dabei auf jede beliebige, dem Fachmann bekannte Art erfolgen. So ist es zum Beispiel möglich, zur Erwärmung des Phosgens einen Wärmetauscher einzusetzen, der genauso aufgebaut ist wie der Wärmetauscher, in dem das Amin verdampft und überhitzt wird. Jedoch ist auch jeder beliebige andere Wärmetauscher zur Erwärmung des Phosgens einsetzbar. Dies ist insbesondere möglich, weil sich das Phosgen bei den Temperaturen, auf die es erwärmt wird, nicht unter Bildung von Ablagerungen zersetzt. Somit ist es nicht erforderlich, das Phosgen besonders schnell zu erhitzen.

Nach dem Mischen werden das Phosgen und das Amin einer Reaktionszone zugeführt. Das Vermischen erfolgt üblicherweise in einer Mischzone. Die Mischzone und die Reaktionszone können dabei aufeinanderfolgende Teile eines Reaktors sein, wie er zur Herstellung von Isocyanaten durch Gasphasenphosgenierung von Aminen eingesetzt wird. Im Allgemeinen wird als Reaktor ein Rohrreaktor verwendet. Im Reaktor wird das Amin mit dem Phosgen zum korrespondierenden Isocyanat und Chlorwasserstoff umgesetzt. Das Phosgen wird dabei üblicherweise im Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Diamine und Diisocyanate können aliphatisch, cycloaliphatisch oder aromatisch sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung "(cyclo)aliphatische Isocyanate" für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylendi(phenylisocyanat) (MDI), 2,4- und/oder 2,6-Toluylendiisocyanat (TDI) und 1,5-oder 1,8-Naphtyldiisocyanat (NDI).

Beispiele für (cyclo)aliphatische Diisocyanate sind aliphatische Diisocyanate wie 1,4-Tetramethylendiisocyanat, 1,6-Hexamethylendüsocyanat (1,6-Diisocyanatohexan), 1,8-Octamethylendiisocyanat, 1,1 0-Decamethylendiisocyanat, 1,12-Dodecamethylen-diisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(bezieh-ungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyanato-3,3,5-trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5-trimethyl-5-(isocyanatomethyl)cyclohexan und 4,4'-Di(isocyanatocyclohexyl)methan.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höhstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IPDA) und 4,4-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphtyldiamin (NDA) und 2,4'- oder 4,4'-Methylen(diphenyldiamin) (MDA) oder Isomerengemische davon. Bevorzugt sind 2,4-und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Isocyanate), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff bilden. Die resultierenden Aminohydrochloride fallen leicht aus und sind nur schwer wieder verdampfbar beziehungsweise zersetzbar.

Neben dem Einsatz eines Rohrreaktors ist es auch möglich, im Wesentlichen quaderförmige Reaktionsräume, beispielsweise Plattenreaktoren, zu verwenden. Auch jeder beliebige andere Querschnitt des Reaktors ist möglich.

Um die Bildung von Nebenprodukten zu vermeiden, ist es bevorzugt, Phosgen im Überschuss zu führen. Um nur den für die Reaktion notwendigen Anteil an Aminen zuzuführen, ist es möglich, das Amin mit einem Inertgas zu mischen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphthalin, Decahydronaphthalin, Kohlenstoffdioxid oder Kohlenstoffmonoxid eingesetzt werden. Bevorzugt werden Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, so dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5, beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgemisch unmittelbar nach der Reaktion abgekühlt. Zum Abkühlen des Reaktionsgemischs wird vorzugsweise ein Quench eingesetzt. Hierbei wird vorzugsweise ein flüssiges Quenchmedium zugegeben. Durch Verdampfung nimmt das Quenchmedium Wärme auf und führt zu einer schnellen Abkühlung des Reaktionsgemischs.

An den Quench können sich weitere Vorrichtungen zur Abkühlung anschließen. So können zum Beispiel weitere Quenche vorgesehen sein. Auch ist es möglich, nach der direkten Abkühlung im Quench eine indirekte Kühlung in einem Wärmetauscher vorzusehen.

Um eine Anreicherung an nicht verdampfbaren Rückständen im Verdampfer, in dem das Amin verdampft und überhitzt wird, zu vermeiden, ist es bevorzugt, aus dem Verdampfer ein gasförmiges Amin sowie einen unverdampfbare Rückstände enthaltenden Spülstrom abzuziehen. Auf diese Weise können unverdampfbare Rückstände ausgeschleust werden. Die unverdampfbaren Rückstände können dabei zum einen im Verdampfer durch Zersetzung und Oligomerisierung des Amins gebildet werden oder auch bereits in den Verdampfer eingebracht werden. Mithilfe des Spülstroms werden die unverdampfbaren Rückstände aus dem Verdampfer abgezogen und können so aus dem Verfahren ausgeschleust werden. Um die unverdampfbaren Rückstände auszuschleusen, jedoch ebenfalls ausgetragenes Amin wieder zurückzugewinnen, ist es bevorzugt, den Spülstrom in eine dem Verdampfer vorgeschaltete Kolonne zur Gewinnung des Amins zurückzuleiten. In der Kolonne wird das Amin vorzugsweise durch ein destillatives Verfahren von anderen Substanzen getrennt. Auf diese Weise kann für die Gasphasenphosgenierung ausreichend aufgereinigtes Amin zur Verfügung gestellt werden.

Die Kolonne, in die der Spülstrom zurückgeleitet wird, ist zum Beispiel Teil einer Anlage zur Herstellung des für die Herstellung des Isocyanats eingesetzten Amins.

Um das Amin zu verdampfen und auf die erforderliche Reaktionstemperatur von 200 bis 400°C zu überhitzen, wird als Heizmedium, das die Rohre durchströmt, vorzugsweise eine Salzschmelze, ein Brenngas oder Wasserdampf eingesetzt. Wenn Wasserdampf eingesetzt wird, so ist dieser auf einen Druck verdichtet, bei dem die Temperatur des Wasserdampfs oberhalb der gewünschten Temperatur für das überhitzte Amin liegt. Neben dem Einsatz von gesättigtem Wasserdampf ist es hierbei auch möglich, als Heizmedium überhitzten Wasserdampf einzusetzen. Aufgrund der zum Erhalten der notwendigen spezifischen Wärmetauscherfläche erforderlichen kleinen Rohrdurchmesser ist auch eine Beheizung des Verdampfers mit einem Gas möglich. Neben überhitztem Wasserdampf können hierbei zum Beispiel auch Abgase aus einer Verbrennung eingesetzt werden.

Neben dem Einsatz der Abgase aus einer Verbrennung, einer Salzschmelze oder von Wasserdampf ist es auch möglich, die Beheizung durch katalytische Verbrennung eines Brennstoffs in den Rohren zu realisieren. In diesem Fall wird ein Brennstoff in die Rohre des Verdampfers eingeleitet und in den Rohren zum Beispiel durch Zugabe von Sauerstoff, beispielsweise in Form von Luft oder auch mit Sauerstoff angereicherter Luft, verbrannt. Die bei der Verbrennung entstehende Wärme wird dann über die Rohrwandungen an das Amin abgegeben, wodurch das Amin verdampft und überhitzt wird. Um eine katalytische Verbrennung des Brennstoffs zu ermöglichen, ist es zum Beispiel möglich, die Innenwandungen der Rohre mit einer katalytisch aktiven Beschichtung zu versehen. Als katalytisch aktive Beschichtung kann dabei jede beliebige, dem Fachmann bekannte katalytisch aktive Beschichtung eingesetzt werden. Als Kata-lysator wird dabei üblicherweise ein Edelmetall, insbesondere ein Edelmetall der Platingruppe, beispielsweise Platin oder Palladium, eingesetzt. Das als Katalysator eingesetzte Metall kann dann zum Beispiel durch ein geeignetes Beschichtungsverfahren, beispielsweise ein Dampfabscheidungsverfahren wie Chemical Vapor Deposition (CVD) oder Physical Vapor Deposition (PVD), an der Innenwand des Rohrs abgeschieden werden. Alternativ ist es jedoch auch möglich, das katalytisch aktive Metall zum Beispiel einer Keramik beizufügen, mit der die Oberflächen der Rohre beschichtet werden. Bevorzugt ist es jedoch, das katalytisch aktive Metall direkt auf die Innenwandung der Rohre aufzubringen.

Neben einer Beschichtung der Innenwandungen mit einer katalytisch aktiven Substanz ist es alternativ auch möglich, den Katalysator zum Beispiel in Form einer Schüttung in den Rohren zu platzieren. In diesem Fall liegt der Katalysator vorzugsweise als feinteiliges Granulat oder als Pulver vor.

Wenn die Verdampfung und Überhitzung des Amins mit einem Brenngas, bevorzugt durch katalytische Verbrennung in den Rohren, erfolgt, wird als Brenngas vorzugsweise Erdgas eingesetzt.

Wenn die Verdampfung des Amins unter Einsatz von Wasserdampf erfolgt, so ist es gegebenenfalls notwendig, dem Verdampfer einen Überhitzer nachzuschalten, um das Amin auf die erforderliche Reaktionstemperatur zu bringen. Dies ist insbesondere dann der Fall, wenn die Temperatur des Wasserdampfs nicht hoch genug ist, um das Amin auf Reaktionstemperatur zu überhitzen. Der dem Verdampfer nachgeschaltete Überhitzer wird dann mit einem von Wasserdampf verschiedenen Heizmedium, beispielsweise einem Brenngas oder auch einer Salzschmelze, geheizt. Der Überhitzer ist dabei vorzugsweise ebenso aufgebaut wie der Verdampfer, wobei sich die Anzahl der Rohre und die Länge der Rohre von Verdampfer und Überhitzer unterscheiden können. Alternativ ist es auch möglich, einen elektrisch beheizten Überhitzer einzusetzen.

Alternativ ist es auch möglich, den Verdampfer zweiteilig zu gestalten, wobei zunächst Rohre eingesetzt werden, die von Wasserdampf durchströmt werden und direkt im Anschluss an die von Wasserdampf durchströmten Rohre Rohre eingesetzt werden, in denen entweder ein Brenngas verbrannt wird oder die von einer Salzschmelze durchströmt werden, um den im Bereich der von Wasserdampf durchströmten Rohre verdampften Aminstrom im nachfolgenden Verdampferteil, der die mit einer Salzschmelze durchströmten Rohre beziehungsweise die Rohre, in denen eine katalytische Verbrennung durchgeführt wird, enthält, überhitzt wird.

Das Aufheizen des Phosgens auf die erforderliche Reaktionstemperatur erfolgt ebenfalls indirekt durch Einsatz eines Heizmediums, beispielsweise Dampf, einer Salzschmelze oder durch Verbrennung eines Brennstoffs oder durch Einsetzen von Abgasen einer Verbrennung als Heizmedium oder aber auch durch Verwendung einer elektrischen Beheizung.

Um auch am Mantel des Verdampfers für das Amin Ablagerungen zu vermeiden, ist es weiterhin bevorzugt, wenn der Mantel des Verdampfers keine Kanten aufweist. Insbesondere ist es bevorzugt, wenn der Mantel des Verdampfers einen kreisförmigen oder einen ovalen Querschnitt aufweist. Der Durchmesser des Verdampfermantels kann dabei beliebig gewählt werden. Die Größe ist hierbei abhängig von der eingesetzten Menge an Amin und der sich daraus ergebenden vom Amin durchströmten Querschnittsfläche. Um die Bildung von Ablagerungen am Mantel zu vermeiden, insbesondere aufgrund einer kalten Mantelfläche, ist es weiterhin möglich, den Mantel des Verdampfers als Doppelmantel auszubilden, wobei dieser ebenfalls von einem Heizmedium durchströmt werden kann. Das Heizmedium ist dabei vorzugsweise das gleiche wie das Heizmedium, das durch die Rohre des Verdampfers strömt.

### Beispiele

### Vergleichsbeispiel

In ein gerührtes Wärmebad aus Diphyl mit einer Temperatur von 350°C wurde eine Kapillare mit 4 mm Innendurchmesser und einer Länge von 1m (Spezifische Wärmeübertragungsfläche 1000 m²/m³) getaucht. In der Kapillare wurden 100 g/h eines Isomerengemisches aus 80% 2,4-TDA und 20% 2,6-TDA mit einer Zulauftemperatur von 130°C bei einem Druck von 3 bar absolut verdampft.

Nach 18,3 Stunden Betriebszeit musste die Anlage wegen Verstopfung der Kapillaren abgestellt werden.

### Beispiel

Durch ein Mantelrohr mit Innendurchmesser 29,5 mm sind koaxial auf einer Länge von 400 mm insgesamt 37 Kapillaren mit einem Außendurchmesser von 3,17 mm geführt. Die Kapillaren sind gleichmäßig über den Querschnitt des Mantelrohrs verteilt und werden mit Heißluft von ca. 600°C durchströmt. In den Mantelraum werden ca. 2 kg/h eines Isomerengemisches aus 80% 2,4-TDA und 20% 2,6-TDA mit einer Zulauftemperatur von 120°C bei einem Druck von ungefähr 4 bar absolut dosiert, im Mantelraum verdampft und auf eine Austrittstemperatur von 430°C überhitzt. Die spezifische Wärmeübergangsfläche beträgt 950 m²/m³.

Trotz der erhöhten Siedetemperatur aufgrund des erhöhten Drucks sowie die zusätzliche Überhitzung auf 430°C im Unterschied zum Vergleichsbeispiel konnte mit der Anlage über einen Zeitraum von mehr als 80 Stunden TDA ohne Verstopfung verdampft werden.

## Patentansprüche

1. Verfahren zur Herstellung von Isocyanaten durch Umsetzung der korrespondierenden Amine mit Phosgen in der Gasphase, gegebenenfalls in Gegenwart eines Inertmediums, folgende Schritte umfassend:
(a) Verdampfen des Amins in einem Verdampfer,
(b) Überhitzen des Amins,
(c) Mischen des gasförmigen Amins mit dem Phosgen und Einleiten in eine Reaktionszone,
(d) Umsetzen von Amin und Phosgen in der Reaktionszone zum Isocyanat, wobei ein Isocyanat und Chlorwasserstoff enthaltendes Reaktionsgemisch gebildet wird,
(e) Abkühlen des Isocyanat und Chlorwasserstoff enthaltenden Reaktionsgemischs,
**dadurch gekennzeichnet, dass** der Verdampfer einen Behälter umfasst, in dem Rohre enthalten sind, die von einem Heizmedium durchströmt werden, wobei Anzahl und Durchmesser der Rohre so ausgeführt sind, dass die Rohre eine spezifische Wärmetauscherfläche bezogen auf das vom Amin durchströmte Volumen von mindestens 300 m²/m³ aufweisen.

2. Verfahren gemäß Anspruch 1, **dadurch gekennzeichnet, dass** die Rohre parallel zur Strömungsrichtung des Amins im Behälter angeordnet sind.

3. Verfahren gemäß Anspruch 1 oder 2, **dadurch gekennzeichnet, dass** die Rohre eine glatte Oberfläche aufweisen.

4. Verfahren gemäß einem der Ansprüche 1 bis 3, **dadurch gekennzeichnet, dass** die Rohre einen kreisförmigen Querschnitt aufweisen.

5. Verfahren gemäß einem der Ansprüche 1 bis 4, **dadurch gekennzeichnet, dass** die Rohre einen Außendurchmesser von maximal 10 mm, bevorzugt von maximal 8 mm aufweisen.

6. Verfahren gemäß einem der Ansprüche 1 bis 5, **dadurch gekennzeichnet, dass** die Rohre eine Länge im Bereich von 0,1 bis 5 m aufweisen.

7. Verfahren gemäß einem der Ansprüche 1 bis 6, **dadurch gekennzeichnet, dass** das Amin in dem Verdampfer verdampft und auf Reaktionstemperatur überhitzt wird und gegebenenfalls vorgeheizt wird.

8. Verfahren gemäß einem der Ansprüche 1 bis 7, **dadurch gekennzeichnet, dass** aus dem Verdampfer ein gasförmiges Amin sowie unverdampfbare Rückstände enthaltender Spülstrom abgezogen wird.

9. Verfahren gemäß Anspruch 8, **dadurch gekennzeichnet, dass** zumindest ein Teil des Spülstroms in eine dem Verdampfer vorgeschaltete Kolonne zur Gewinnung des Amins zurückgeleitet wird.

10. Verfahren gemäß einem der Ansprüche 1 bis 9, **dadurch gekennzeichnet, dass** das die Rohre durchströmende Heizmedium eine Salzschmelze, ein Brenngas oder Wasserdampf ist.

11. Verfahren gemäß einem der Ansprüche 1 bis 10, **dadurch gekennzeichnet, dass** die Beheizung durch katalytische Verbrennung eines Brennstoffs in den Rohren erfolgt.

12. Verfahren gemäß Anspruch 11, **dadurch gekennzeichnet, dass** die Rohre an ihren Innenwandungen mit einer katalytisch aktiven Beschichtung versehen sind.

## Claims

1. A process for preparing isocyanates by reacting the corresponding amines with phosgene in the gas phase, in the presence or absence of an inert medium, which comprises the following steps:
(a) vaporization of the amine in a vaporizer,
(b) superheating of the amine,
(c) mixing of the gaseous amine with the phosgene and introduction into a reaction zone,
(d) reaction of amine and phosgene to give isocyanate in the reaction zone, with a reaction mixture comprising isocyanate and hydrogen chloride being formed,
(e) cooling of the reaction mixture comprising isocyanate and hydrogen chloride,
wherein the vaporizer comprises a vessel in which tubes through which a heating medium flows are comprised, where number and diameter of the tubes are designed so that the tubes have a specific heat transfer area based on the volume through which the amine flows of at least 300 m²/m³.

2. The process according to claim 1, wherein the tubes are arranged parallel to the flow direction of the amine in the vessel.

3. The process according to claim 1 or 2, wherein the tubes have a smooth surface.

4. The process according to any of claims 1 to 3, wherein the tubes have a circular cross section.

5. The process according to any of claims 1 to 4, wherein the tubes have an external diameter of not more than 10 mm, preferably not more than 8 mm.

6. The process according to any of claims 1 to 5, wherein the tubes have a length in the range from 0.1 to 5 m.

7. The process according to any of claims 1 to 6, wherein the amine is vaporized and superheated to the reaction temperature and is optionally preheated in the vaporizer.

8. The process according to any of claims 1 to 7, wherein a purge stream comprising gaseous amine and nonvolatile residues is taken off from the vaporizer.

9. The process according to claim 8, wherein at least part of the purge stream is recirculated to a column for isolating the amine located upstream of the vaporizer.

10. The process according to any of claims 1 to 9, wherein the heating medium flowing through the tubes is a salt melt, a fuel gas or steam.

11. The process according to any of claims 1 to 10, wherein heating is effected by catalytic combustion of a fuel in the tubes.

12. The process according to claim 11, wherein the tubes are provided on their interior walls with a catalytically active coating.

## Revendications

1. Procédé pour la préparation d'isocyanates par transformation des amines correspondantes avec du phosgène en phase gazeuse, le cas échéant en présence d'un milieu inerte, comprenant les étapes suivantes :
(a) évaporation de l'amine dans un évaporateur,
(b) surchauffage de l'amine,
(c) mélange de l'amine gazeuse avec le phosgène et introduction dans une zone de réaction,
(d) transformation de l'amine et du phosgène en isocyanate dans la zone de réaction, un mélange réactionnel contenant de l'isocyanate et du chlorure d'hydrogène étant formé,
(e) refroidissement du milieu réactionnel contenant de l'isocyanate et du chlorure d'hydrogène,
**caractérisé en ce que** l'évaporateur comprend un récipient, qui contient des tubes, au travers desquels s'écoule un agent chauffant, le nombre et le diamètre des tubes étant réalisés de manière telle que les tubes présentent une surface spécifique d'échange thermique, par rapport au volume traversé par l'amine, d'au moins 300 m²/m³.

2. Procédé selon la revendication 1, **caractérisé en ce que** les tubes sont disposés parallèlement au sens d'écoulement de l'amine dans le récipient.

3. Procédé selon la revendication 1 ou 2, **caractérisé en ce que** les tubes présentent une surface lisse.

4. Procédé selon l'une quelconque des revendications 1 à 3, **caractérisé en ce que** les tubes présentent une section transversale circulaire.

5. Procédé selon l'une quelconque des revendications 1 à 4, **caractérisé en ce que** des tubes présentent un diamètre externe d'au maximum 10 mm, de préférence d'au maximum 8 mm.

6. Procédé selon l'une quelconque des revendications 1 à 5, **caractérisé en ce que** les tubes présentent une longueur dans la plage de 0,1 à 5 m.

7. Procédé selon l'une quelconque des revendications 1 à 6, **caractérisé en ce que** l'amine est évaporée dans l'évaporateur et surchauffée à la température de réaction et le cas échéant préchauffée.

8. Procédé selon l'une quelconque des revendications 1 à 7, **caractérisé en ce qu'**un flux de rinçage contenant de l'amine gazeuse ainsi que des résidus qui ne peuvent être évaporés est soutiré de l'évaporateur.

9. Procédé selon la revendication 8, **caractérisé en ce qu'**au moins une partie du flux de rinçage est recyclée dans une colonne disposée en amont de l'évaporateur pour produire l'amine.

10. Procédé selon l'une quelconque des revendications 1 à 9, **caractérisé en ce que** l'agent chauffant qui s'écoule au travers des tubes est un sel fondu, un gaz de combustion ou de la vapeur d'eau.

11. Procédé selon l'une quelconque des revendications 1 à 10, **caractérisé en ce que** le chauffage est réalisé par combustion catalytique d'un combustible dans les tubes.

12. Procédé selon la revendication 11, **caractérisé en ce que** les tubes sont pourvus d'un revêtement catalytiquement actif sur leurs parois internes.
